# EUROPEAN PATENT SPECIFICATION

(11) **EP 3 932 407 B1**
(45) Date of publication and mention of the grant of the patent: **18.10.2023**
(21) Application number: 19917480.6
(22) Date of filing: 31.07.2019
(51) Int. Cl.: A61K 31/4965, A61K 9/24, A61P 9/10

(54) **ENTERIC COATED TABLET COMPOSITION COMPRISING TETRAMETHYLPYRAZINE NITRONE**
ENTERISCH UMMANTELTE TABLETTENZUSAMMENSETZUNG ENTHALTEN TETRAMETHYLPYRAZINNITRON
COMPOSITION DE COMPRIME A ENROBAGE ENTERIQUE COMPRENANT DE LA TETRAMETHYLPYRAZINE NITRONE

(30) Priority: 27.02.2019 CN 201910143739
(43) Date of publication of application: 05.01.2022
(73) Proprietor: Guangzhou Magpie Pharmaceuticals Co., Ltd., Guangzhou, Guangdong 510700 (CN)
(72) Inventor: LIU, Wei, Guangzhou, Guangdong 510700 (CN); SUN, Yewei, Guangzhou, Guangdong 510700 (CN); WANG, Yuqiang, Guangzhou, Guangdong 510700 (CN)
(74) Representative: Cohausz & Florack
(86) International application number: PCT/CN2019/098709
(87) International publication number: WO 2020/173046

(56) References cited:
- WO-A1-2009/129726
- CN-A- 1 683 345
- CN-A- 104 922 086
- CN-A- 105 963 298
- CN-A- 107 417 631
- CN-A- 108 815 128
- LI, Sai et al.: "Concentration of Nitrone Derivative of Tetramethylpyrazine in Brain Tissue of Cerebral Ischemia Model Rats", The Chinese Journal of Clinical Pharmacology, vol. 27, no. 6, 30 June 2011 (2011-06-30), XP009522914, ISSN: 1007-4406
- LU, Huifen: "Formula Screening and Evaluation of Rabeprazole Sodium Enteric-coated Tablet", Hebei Journal of Industrial Science and Technology, vol. 33, no. 4, 31 July 2016 (2016-07-31), pages 314-318, XP009522918, ISSN: 1008-1534

## Description

### TECHNICAL FIELD

The present invention relates to the technical field of pharmaceutical preparations, and particularly to a pharmaceutical composition comprising TBN, or a salt or a hydrate thereof, and a preparation method therefor.

### BACKGROUND

At present, no specific drugs for the treatment of stroke are available in clinic, and most of the drugs on the market fail to meet the requirements because of poor efficacy or large toxic side effects.

The nitrone compound of the present invention is a nitrone compound TBN that is structurally modified TMP, and has a chemical name of (cis)-2-methyl-N-((3,5,6-trimethylpyrazin-2-yl)methylene)2-propylamine oxide, and a chemical structure shown below:

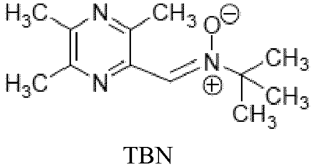

TBN has improved antioxidation ability while maintaining the thrombolytic ability, and can be used clinically for the treatment of neurodegenerative diseases, cardiovascular and cerebrovascular diseases, etc. WO 2009/129726 describes the synthesis of tetramethylpyrazine nitrone (TBN). Possible pharmaceutical formulations comprising TBN, like e.g. a tablet, are listed.

### SUMMARY

An object of the present invention is to provide a pharmaceutical composition comprising TBN, or a salt or a hydrate thereof. TBN is a new chemical drug having an absolutely new structure and great development prospects.

The present invention provides a pharmaceutical composition comprising TBN, or a salt or a hydrate thereof. The pharmaceutical composition comprises:
(1) a tablet core comprising the active ingredient TBN or a pharmaceutically acceptable salt or hydrate thereof and an alkalizing agent; and
(2) an enteric layer outside the tablet core, comprising an opaquer and a coating material.

In the composition of the present invention, the tablet core may further comprise a binder and/or a disintegrant and/or a filler and/or a lubricant; and the enteric layer may further include a plasticizer and/or an anti-sticking agent.

In the composition of the present invention, an isolation layer may be further provided between the tablet core and the enteric layer, and further preferably, a moisture barrier may be further provided between the isolation layer and the enteric layer.

Further preferably, the isolation layer comprises a coating and an anti-sticking agent.

Further preferably, the enteric layer of the present invention accounts for 0.5-20%, preferably 1-15%, and more preferably 1-11% by weight (based on the weight of the tablet core, based on the total weight of the tablet core and the isolation layer where the isolation layer is present, or based on the total weight of the tablet core, the isolation layer and the moisture barrier where the isolation layer and the moisture barrier are present).

Further preferably, the alkalizing agent of the present invention may be one or more selected from sodium bicarbonate, magnesia and magnesium carbonate. To improve the stability of the active ingredient, the alkalizing agent of the present invention is preferably sodium bicarbonate. The inventor finds that compared with other alkalizing agents, sodium bicarbonate can further improve the stability of the active ingredient. Especially under high temperature and humidity conditions, the stabilizing effect is significantly better than other alkalizing agents.

Further preferably, the weight ratio of the active ingredient to the alkalizing agent in the present invention is (90-110): (5-30), and preferably (90-110): (10-25).

Further preferably, the opaquer of the present invention may be an opaquer commonly used in the art. To improve the stability of the active ingredient, the opaquer of the present invention is titania.

The coating material in the enteric layer of the present invention may be one or more of methacrylic acid-ethyl acrylate copolymer, hydroxypropyl methylcellulose phthalate (HPMCP, HP-55), hydroxypropyl methylcellulose acetate succinate (HPMCAS, AS-HG), hydroxypropyl methylcellulose acetate succinate (HPMCAS, AS-LG), Eudragit^{®} L30D-55, Eudragit^{®} L100, and Eudragit^{®} NE30D, and preferably one or more of methacrylic acid-ethyl acrylate copolymer, hydroxypropyl methylcellulose phthalate (HPMCP, HP-55), and hydroxypropyl methylcellulose acetate succinate (HPMCAS, AS-HG).

Further preferably, the weight ratio of the opaquer to the coating material in the present invention is (0.5-2.5): (5-20), and preferably 1: (10-20).

The binder of the present invention may be one or more selected from hydroxypropyl cellulose, Polyvidone K30, hydroxymethyl cellulose, methyl cellulose, hydroxyethyl cellulose, carboxymethyl cellulose, polyvinylpyrrolidone, and preferably hydroxypropyl cellulose or Polyvidone K30.

Further preferably, the weight ratio of the active ingredient to the binder in the present invention is (90-110): (5-30), and preferably (90-110): (10-30). The inventor finds that when the binder content is lower than this mixing ratio, the edge of the tablet core is easy to wear, causing failed coating.

The disintegrant of the present invention may be one or more selected from Crospovidone, Croscarmellose sodium, Carboxymethyl starch sodium (CMS), sodium hydroxypropyl starch or low-substituted hydroxypropyl cellulose, and preferably Crospovidone or Carboxymethyl starch sodium.

Further preferably, the weight ratio of the active ingredient to the disintegrant in the present invention is (90-110): (3-30), and preferably (90-110): (5-25).

The filler of the present invention may be one or more selected from mannitol, microcrystalline cellulose, lactose, xylitol, sucrose, glucose, sorbitol, starch, pregelatinized starch, calcium sulfate, calcium carbonate, calcium hydrogen phosphate or light magnesia; preferably one or more selected from mannitol, microcrystalline cellulose, lactose or pregelatinized starch; and further preferably mannitol or pregelatinized starch.

Further preferably, the weight ratio of the active ingredient to the filler in the present invention is (90-110):(60-200), preferably (90-110):(70-150), and further preferably (90-110):(75-140).

The lubricant of the present invention may be one or more selected from magnesium stearate, stearic acid, talc, hydrogenated vegetable oil, glyceryl behenate or micronized silica gel, and preferably magnesium stearate.

Further preferably, the weight ratio of the active ingredient to the lubricant in the present invention is (90-110):(0.2-2), and preferably (90-110):(0.5-1.5).

In a preferred embodiment of the present invention, the tablet core of the present invention comprises the active ingredient TBN or a pharmaceutically acceptable salt or hydrate thereof, an alkalizing agent, a binder, a disintegrant, a filler and a lubricant, where the weight ratio of the active ingredient alkalizing agent:binder:disintegrant:filler:lubricant is (90-110):(5-30):(5-30):(3-30):(60-200):(0.2-2), and preferably (90-110):(10-25):(10-30):(5-25):(70-150):(0.5-1.5).

The plasticizer in the enteric layer of the present invention may be one or more of triethyl citrate, PEG4000, PEG6000, triethyl acetylcitrate, and polysorbate-80. Preferably, the weight ratio of the opaquer to the plasticizer in the enteric layer is 1 :0.5-10, and preferably 1 :0.5-7.

The anti-sticking agent in the enteric layer of the present invention may be one or more of talc, glyceryl monostearate, and micronized silica gel. Preferably, the weight ratio of the opaquer to the anti-sticking agent in the enteric layer is 1:0.5-10, and preferably 1:0.5-5.

In a preferred embodiment of the present invention, the enteric layer of the present invention comprises an opaquer, a coating material, a plasticizer and an anti-sticking agent, where the weight ratio of the opaquer, the coating material, the plasticizer and the anti-sticking agent can be as described above.

Further preferably, the isolation layer of the present invention accounts for 2-15%, and preferably 2-10% by weight (based on the weight of the tablet core).

The isolation layer of the present invention may include a coating material and/or an anti-sticking agent in the isolation layer.

The coating material in the isolation layer of the present invention may be one or more of hydroxypropyl cellulose or ethyl cellulose.

The anti-sticking agent used in the isolation layer of the present invention may be one or more of talc, magnesia, glyceryl monostearate, and micronized silica gel, and preferably talc or magnesia.

In a preferred embodiment of the present invention, the isolation layer of the present invention comprises a coating material and an anti-sticking agent in the isolation layer, where the weight ratio of the coating material to the anti-sticking agent in the isolation layer is (1-10): 1, and preferably (1-5): 1.

The moisture barrier material of the present invention is Opadry, such as one or more of Opadry (81W680001) or Opadry (21K58794). Preferably, the moisture barrier accounts for 3-5% by weight (based on the weight of the tablet core, or based on the total weight of the tablet core and the isolation layer where the isolation layer is present).

The composition of the present invention can be prepared according to the commonly used preparation method of enteric-coated tablets in the prior art. In one embodiment of the present invention, a method for preparing a pharmaceutical composition comprising TBN, or a salt or a hydrate thereof is also provided. The preparation method includes the following steps:
(1) sieving the active ingredient TBN or a pharmaceutically acceptable salt or hydrate thereof, and an alkalizing agent and/or a binder and/or a disintegrant and/or a filler, directly mixing or mixing and then granulating, optionally mixing with a lubricant, and tableting to obtain tablet cores; and
(2) coating the tablet core in Step (1) with an enteric layer at 40-50°C and removing to obtain a TBN enteric-coated tablet.

Before the coating the tablet core in Step (1) with an enteric layer at 40-50°C in Step (2) of the present invention, the tablet core can be coated with the isolation layer at 45-65°C, removed, and then coated with the enteric layer at 40-50°C.

The composition or weight ratio of the tablet core, the isolation layer, and the enteric layer, etc. are as described above.

In the preparation process of the pharmaceutical composition of the present invention, the technical schemes that are not described in detail are conventional technical schemes in the art. For example, the granulation in Step (1) may be dry granulation commonly used in the art; and the mixing time in Step (1) is a conventional mixing time. For example, in an embodiment of the present invention, the mixing time is 20-40 min, and the mixing method may be a conventional method using, for example, a hopper mixer.

The present invention also provides use of the pharmaceutical composition comprising TBN, or a salt or a hydrate thereof, in the treatment of neurodegenerative diseases and cardiovascular and cerebrovascular diseases.

The neurodegenerative diseases mentioned the present invention may include, but are not limited to: epilepsy, Parkinson's disease, Huntington's disease, amyotrophic (spinal) lateral sclerosis, Alzheimer's disease, and multiple sclerosis, etc.

The cardiovascular and cerebrovascular diseases mentioned in the present invention may include, but are not limited to: stroke, myocardial ischemia or reperfusion injury, myocarditis, atherosclerosis, cardiopulmonary lateral flow, respiratory distress syndrome, chronic obstructive pulmonary disease, coronary heart disease or sudden heart attack, etc.

The "pharmaceutically acceptable salt" mentioned the present invention means those salts that retain the biological effectiveness and properties of the parent compound. Such salts include: salts with acids obtained through reaction of the parent compound as a free base with inorganic acids including hydrochloric acid, hydrobromic acid, nitric acid, phosphoric acid, metaphosphoric acid, sulfuric acid, sulfurous acid and perchloric acid; or organic acids including acetic acid, trifluoroacetic acid, propionic acid, acrylic acid, caproic acid, cyclopentylpropionic acid, glycolic acid, pyruvic acid, oxalic acid, (D) or (L) malic acid, fumaric acid, maleic acid, benzoic acid, hydroxybenzoic acid, γ-hydroxybutyric acid, methoxybenzoic acid, phthalic acid, methanesulfonic acid, ethanesulfonic acid, naphthalene-1-sulfonic acid, naphthalene-2-sulfonic acid, p-toluene sulfonic acid, salicylic acid, tartaric acid, citric acid, lactic acid, cinnamic acid, dodecylsulfuric acid, gluconic acid, glutamic acid, aspartic acid, stearic acid, mandelic acid, succinic acid or malonic acid, etc.

The present invention has the following beneficial effects.
(1) The pharmaceutical composition of the present invention can significantly improve the stability of the active ingredient.
(2) The pharmaceutical composition of the present invention can increase the bioavailability of TBN in the body, and the bioavailability of the enteric-coated tablets is increased by more than 2 times compared with TBN (API, active pharmaceutical ingredient).
(3) The pharmaceutical composition according to the present invention has the enteric layer with good film-forming ability and is easy for large-scale industrial production.

### BRIEF DESCRIPTION OF THE DRAWINGS

Fig. 1 shows the drug concentration-time curve after oral administration of enteric-coated tablets to beagle dogs.
Fig. 2 shows the drug concentration-time curve after oral administration of TBN (API) to beagle dogs.

### DETAILED DESCRIPTION

The following examples are provided for a better understanding of the present invention; however, the present invention is not limited thereto. The methods given in examples below are all conventional methods, unless it is otherwise stated. Test materials used in the following examples are commercially available, unless otherwise specified.

### Example 1

TBN (API), mannitol, pregelatinized starch, Crospovidone, sodium bicarbonate and magnesium stearate were sieved through a 40-mesh screen for later use. 100 g of TBN (API), 70 g of mannitol, 70 g of pregelatinized starch, 7.5 g of Polyvidone K30, 5 g of Crospovidone, and 10 g of sodium bicarbonate were weighed, and mixed for 30 min in a hopper mixer in the laboratory. Then, 1 g of magnesium stearate was added and mixed for another 1 min. The resulting material was directly tableted, to obtain 1000 tablet cores with a specification of 100 mg and an average tablet weight of 0.2635 g.

### Example 2

TBN (API), mannitol, pregelatinized starch, Crospovidone, sodium bicarbonate, and magnesium stearate were sieved through a 40-mesh screen for later use. 100 g of TBN (API), 70 g of mannitol, 70 g of pregelatinized starch, 7.5 g of Polyvidone K30, 5 g of Crospovidone, and 20 g of sodium bicarbonate were weighed, and mixed for 30 min in a hopper mixer in the laboratory. Then, 1 g of magnesium stearate was added and mixed for another 1 min. The resulting material was directly tableted, to obtain 1000 tablet cores with a specification of 100 mg and an average tablet weight of 0.2735 g.

### Example 3

TBN (API), mannitol, pregelatinized starch, Crospovidone, magnesia, and magnesium stearate were sieved through a 40-mesh screen for later use. 100 g of TBN (API), 70 g of mannitol, 70 g of pregelatinized starch, 7.5 g of Polyvidone K30, 5 g of Crospovidone, and 20 g of magnesia were weighed, and mixed for 30 min in a hopper mixer in the laboratory. Then, 1 g of magnesium stearate was added and mixed for another 1 min. The resulting material was directly tableted, to obtain 1000 tablet cores with a specification of 100 mg and an average tablet weight of 0.2735 g.

### Example 4

TBN (API), mannitol, pregelatinized starch, Crospovidone, magnesium carbonate and magnesium stearate were sieved through a 40-mesh screen for later use. 100 g of TBN (API), 70 g of mannitol, 70 g of pregelatinized starch, 7.5 g of Polyvidone K30, 5 g of Crospovidone, and 20 g of magnesium carbonate were weighed, and mixed for 30 min in a hopper mixer in the laboratory. Then, 1 g of magnesium stearate was added and mixed for another 1 min. The resulting material was directly tableted, to obtain 1000 tablet cores with a specification of 100 mg and an average tablet weight of 0.2735 g.

### Example 5

TBN (API), mannitol, pregelatinized starch, Crospovidone, sodium bicarbonate and magnesium stearate were sieved through a 40-mesh screen for later use. 100 g of TBN (API), 70 g of mannitol, 70 g of pregelatinized starch, 15 g of Polyvidone K30, 5 g of Crospovidone, and 20 g of sodium bicarbonate were weighed, and mixed for 30 min in a hopper mixer in the laboratory. Then, 1 g of magnesium stearate was added and mixed for another 1 min. The resulting material was directly tablet, to obtain 1000 tablet cores with a specification of 100 mg and an average tablet weight of 0.281 g.

### Example 6

TBN (API), mannitol, pregelatinized starch, Crospovidone, sodium bicarbonate and magnesium stearate were sieved through a 40-mesh screen for later use. 100 g of TBN (API), 60 g of mannitol, 60 g of pregelatinized starch, 30 g of Polyvidone K30, 5 g of Crospovidone, and 20 g of sodium bicarbonate were weighed, and mixed for 30 min in a hopper mixer in the laboratory. Then, 1 g of magnesium stearate was added and mixed for another 1 min. The resulting material was directly tablet, to obtain 1000 tablet cores with a specification of 100 mg and an average tablet weight of 0.276 g.

### Example 7

TBN (API), mannitol, hydroxypropyl cellulose, Crospovidone, sodium bicarbonate and magnesium stearate were sieved through a 40-mesh screen for later use. 300 g of TBN (API), 231 g of mannitol, 75 g of hydroxypropyl cellulose, 51 g of Crospovidone, and 60 g of sodium bicarbonate were weighed, and mixed for 30 min in a hopper mixer in the laboratory. Then, 3 g of magnesium stearate was added and mixed for another 1 min. The resulting material was directly tableted, to obtain 1000 tablet cores with a specification of 300 mg and an average tablet weight of 0.72 g.

### Example 8

TBN (API), mannitol, hydroxypropyl cellulose, Crospovidone, sodium bicarbonate and magnesium stearate were sieved through a 40-mesh screen for later use. 300 g of raw TBN (API), 248.4 g of mannitol, 57.6 g of hydroxypropyl cellulose, 51 g of Crospovidone, and 60 g of sodium bicarbonate were weighed, and mixed for 30 min in a hopper mixer in the laboratory. Then, 3 g of magnesium stearate was added and mixed for another 1 min. The resulting material was directly tableted, to obtain 1000 tablet cores with a specification of 300 mg and an average tablet weight of 0.72 g.

### Example 9

TBN (API), mannitol, hydroxypropyl cellulose, Crospovidone, sodium bicarbonate and magnesium stearate were sieved through a 40-mesh screen for later use. 300 g of TBN (API), 248.4 g of mannitol, 57.6 of hydroxypropyl cellulose, 51 g of Crospovidone, and 60 g of sodium bicarbonate were weighed, mixed for 30 min in a hopper mixer in the laboratory, and dry granulated. Then, 3 g of magnesium stearate was added and mixed for another 1 min, to obtain 1000 tablet cores with a specification of 300 mg and an average tablet weight of 0.72 g.

### Example 10

TBN (API), mannitol, Carboxymethyl starch sodium, sodium bicarbonate, hydroxypropyl cellulose and magnesium stearate were sieved through a 40-mesh screen for later use. 300 g of TBN (API), 272 g of mannitol, 75 g of hydroxypropyl cellulose, 40 g of Carboxymethyl starch sodium, and 60 g of sodium bicarbonate were weighed, and mixed for 30 min in a hopper mixer in the laboratory. Then, 3 g of magnesium stearate was added and mixed for another 1 min. The resulting material was directly tableted, to obtain 1000 tablet cores with a specification of 300 mg and an average tablet weight of 0.75 g.

### Example 11

TBN (API), mannitol, Crospovidone, sodium bicarbonate, hydroxypropyl cellulose and magnesium stearate were sieved through a 40-mesh screen for later use. 300 g of TBN (API), 272 g of mannitol, 75 g of hydroxypropyl cellulose, 40 g of Crospovidone, and 60 g of sodium bicarbonate were weighed, and mixed for 30 min in a hopper mixer in the laboratory. Then, 3 g of magnesium stearate was added and mixed for another 1 min. The resulting material was directly tableted, to obtain 1000 tablet cores with a specification of 300 mg and an average tablet weight of 0.75 g.

### Example 12

TBN (API), mannitol, Crospovidone, sodium bicarbonate, hydroxypropyl cellulose and magnesium stearate were sieved through a 40-mesh screen for later use. 300 g of TBN (API), 231 g of mannitol, 75 g of hydroxypropyl cellulose, 51 g of Crospovidone, and 60 g of sodium bicarbonate were weighed, and mixed for 30 min in a hopper mixer in the laboratory. Then, 3 g of magnesium stearate was added and mixed for another 1 min. The resulting material was directly tableted, to obtain 1000 tablet cores with a specification of 300 mg and an average tablet weight of 0.72 g.

### Example 13

TBN (API), mannitol, pregelatinized starch, Crospovidone, sodium bicarbonate and magnesium stearate were sieved through a 40-mesh screen for later use. 100 g of TBN (API), 60 g of mannitol, 60 g of pregelatinized starch, 30 g of Polyvidone K30, 5 g of Crospovidone, and 10 g of sodium bicarbonate were weighed, and mixed for 30 min in a hopper mixer in the laboratory. Then, 1 g of magnesium stearate was added and mixed for another 1 min. The resulting material was directly tableted.

Then the tablet cores were added to a high-efficiency coating machine, and the prepared isolation coating solution containing 4.81 g of hydroxypropyl cellulose, 4.81 g of talc, and 70.55 g of absolute ethanol was added. The isolation layer accounted for 3.62% by weight. Next, a moisture barrier forming solution containing 12.68 g of Opadry, and 66.57 g of pure water was added. The moisture barrier accounted for 4.60% by weight. After that, an enteric coating solution containing 30.76 g of hydroxypropyl methylcellulose phthalate, 2.46 g of triethyl citrate, 1.54 g of titania, 5.42 g of talc, 275.30 g of absolute ethanol, and 68.83 g of pure water was added, and the enteric layer accounted for 10.67% by weight. Thus, TBN enteric-coated tablets were obtained.

### Example 14

TBN (API), mannitol, pregelatinized starch, Crospovidone, sodium bicarbonate and magnesium stearate were sieved through a 40-mesh screen for later use. 100 g of TBN (API), 60 g of mannitol, 60 g of pregelatinized starch, 30 g of Polyvidone K30, 5 g of Crospovidone, and 10 g of sodium bicarbonate were weighed, and mixed for 30 min in a hopper mixer in the laboratory. Then, 1 g of magnesium stearate was added and mixed for another 1 min. The resulting material was directly tableted.

Then the tablet cores were added to a high-efficiency coating machine, and the prepared isolation coating solution containing 6.73 g of hydroxypropyl cellulose, 4.81 g of talc, and 100.89 g of absolute ethanol was added. The isolation layer accounted for 2.95% by weight. Next, a moisture barrier forming solution containing 9.58 g of Opadry and 50.32 g of pure water was added. The moisture barrier accounted for 3.50% by weight. After that, an enteric coating solution containing 25.74 g of hydroxypropyl methylcellulose phthalate, 2.06 g of triethyl citrate, 1.29 g of titania, 4.70 g of talc, 230.33 g of absolute ethanol, and 57.58 g of pure water was added, and the enteric layer accounted for 9.08% by weight. Thus, TBN enteric-coated tablets were obtained.

### Example 15

TBN (API), mannitol, Crospovidone, sodium bicarbonate, hydroxypropyl cellulose and magnesium stearate were sieved through a 40-mesh screen for later use. 300 g of TBN (API), 248.4 g of mannitol, 57.6 g of hydroxypropyl cellulose, 51 g of Crospovidone, and 60 g of sodium bicarbonate were weighed, mixed for 30 min in a hopper mixer in the laboratory, and granulated. Then, 3 g of magnesium stearate was added and mixed for another 1 min, to obtain tablet cores. Then the tablet cores were added to a high-efficiency coating machine, and the prepared isolation coating solution containing 55.6 g of hydroxypropyl cellulose and 16.4 g of talc was added. The isolation layer accounted for 10% by weight. The composition of the enteric layer was the same as that in Example 24, and the enteric layer accounted for 6% by weight. Thus, TBN enteric-coated tablets were obtained.

### Example 16

TBN (API), mannitol, Crospovidone, sodium bicarbonate, hydroxypropyl cellulose and magnesium stearate were sieved through a 40-mesh screen for later use. 300 g of TBN (API), 248.4 g of mannitol, 57.6 g of hydroxypropyl cellulose, 51 g of Crospovidone, and 60 g of sodium bicarbonate were weighed, mixed for 30 min in a hopper mixer in the laboratory, and granulated. Then, 3 g of magnesium stearate was added and mixed for another 1 min, to obtain tablet cores. Then the tablet cores were added to a high-efficiency coating machine, and the prepared isolation coating solution containing 27.8 g of hydroxypropyl cellulose and 8.2 g of talc was added. The isolation layer accounted for 5% by weight. The composition of the enteric layer was the same as that in Example 24, and the enteric layer accounted for 6% by weight. Thus, TBN enteric-coated tablets were obtained.

### Example 17

TBN (API), mannitol, Crospovidone, sodium bicarbonate and magnesium stearate were sieved through a 40-mesh screen for later use. 100 g of TBN (API), 100 g of mannitol, 10 g of Polyvidone K30, 25 g of Crospovidone, and 60 g of sodium bicarbonate were weighed, and mixed for 30 min in a hopper mixer in the laboratory. Then, 1 g of magnesium stearate was added and mixed for another 1 min. The resulting material was directly tableted.

Then the tablet cores were added to a high-efficiency coating machine, and the prepared isolation coating solution containing 8.78 g of hydroxypropyl cellulose, 8.78 g of talc, and 128.77 g of absolute ethanol was added. The isolation layer accounted for 5.93% by weight. Next, a moisture barrier forming solution containing 14.49 g of Opadry (81W680001), and 76.07 g of pure water was added. The moisture barrier accounted for 4.62% by weight. After that, an enteric coating solution containing 27.39 g of hydroxypropyl methylcellulose phthalate, 2.19 g of triethyl citrate, 1.37 g of titania, 5.00 g of talc, 245.14 g of absolute ethanol, and 61.29 g of pure water was added, and the enteric layer accounted for 8.35% by weight. Thus, TBN enteric-coated tablets were obtained.

### Example 18

TBN (API), mannitol, Crospovidone, sodium bicarbonate and magnesium stearate were sieved through a 40-mesh screen for later use. 100 g of TBN (API), 100 g of mannitol, 10 g of Polyvidone K30, 25 g of Crospovidone, and 60 g of sodium bicarbonate were weighed, and mixed for 30 min in a hopper mixer in the laboratory. Then, 1 g of magnesium stearate was added and mixed for another 1 min. The resulting material was directly tableted.

Then the tablet cores were added to a high-efficiency coating machine, and the prepared isolation coating solution containing 8.78 g of hydroxypropyl cellulose, 8.78 g of talc, and 128.77 g of absolute ethanol was added. The isolation layer accounted for 5.93% by weight. Next, a moisture barrier forming solution containing 6.33 g of Opadry (21K58794), 14.88 g of pure water and 84.29 g of absolute ethanol was added. The moisture barrier accounted for 2.02% by weight. After that, an enteric coating solution containing 27.39 g of hydroxypropyl methylcellulose phthalate, 2.19 g of triethyl citrate, 1.37 g of titania, 5.00 g of talc, 245.07 g of absolute ethanol, and 61.27 g of pure water was added, and the enteric layer accounted for 8.56% by weight. Thus, TBN enteric-coated tablets were obtained.

### Example 19

TBN (API), mannitol, Crospovidone, sodium bicarbonate and magnesium stearate were sieved through a 40-mesh screen for later use. 100 g of TBN (API), 100 g of mannitol, 10 g of Polyvidone K30, 25 g of Crospovidone, and 60 g of sodium bicarbonate were weighed, and mixed for 30 min in a hopper mixer in the laboratory. Then, 1 g of magnesium stearate was added and mixed for another 1 min. The resulting material was directly tableted.

Then the tablet cores were added to a high-efficiency coating machine, and the prepared isolation coating solution containing 8.78 g of hydroxypropyl cellulose, 8.78 g of talc, and 128.77 g of absolute ethanol was added. The isolation layer accounted for 5.93% by weight. Next, a moisture barrier forming solution containing 14.49 g of Opadry, and 76.07 g of pure water was added. The moisture barrier accounted for 4.62% by weight. After that, an enteric coating solution containing 27.39 g of hydroxypropyl methylcellulose phthalate, 2.19 g of triethyl citrate, 1.37 g of titania, 5.00 g of talc, 245.14 g of absolute ethanol, and 61.29 g of pure water was added, and the enteric layer accounted for 8.35% by weight. Thus, TBN enteric-coated tablets were obtained.

### Example 20

TBN (API), mannitol, Crospovidone, sodium bicarbonate and magnesium stearate were sieved through a 40-mesh screen for later use. 100 g of TBN (API), 100 g of mannitol, 10 g of Polyvidone K30, 25 g of Crospovidone, and 60 g of sodium bicarbonate were weighed, and mixed for 30 min in a hopper mixer in the laboratory. Then, 1 g of magnesium stearate was added and mixed for another 1 min. The resulting material was directly tableted.

Then the tablet cores were added to a high-efficiency coating machine, and the prepared isolation coating solution containing 8.78 g of hydroxypropyl cellulose, 8.78 g of talc, and 128.77 g of absolute ethanol was added. The weight was increased by 5.93%. Next, a moisture barrier forming solution containing 14.49 g of Opadry, and 76.07 g of pure water was added. The weight was increased by 4.62%. After that, an enteric coating solution containing 28.31 g of hydroxypropyl methylcellulose acetate succinate (HPMCAS, AS-HG), 2.26 g of triethyl citrate, 1.42 g of titania, 5.17 g of talc, 63.34 g of absolute ethanol, and 253.37 of pure water was added, and the enteric layer accounted for 8.63% by weight. Thus, TBN enteric-coated tablets were obtained.

### Example 21

TBN (API), mannitol, Crospovidone, sodium bicarbonate and magnesium stearate were sieved through a 40-mesh screen for later use. 100 g of TBN (API), 100 g of mannitol, 10 g of Polyvidone K30, 25 g of Crospovidone, and 60 g of sodium bicarbonate were weighed, and mixed for 30 min in a hopper mixer in the laboratory. Then, 1 g of magnesium stearate was added and mixed for another 1 min. The resulting material was directly tableted.

Then the tablet cores were added to a high-efficiency coating machine, and the prepared isolation coating solution containing 8.78 g of hydroxypropyl cellulose, 8.78 g of talc, and 128.77 g of absolute ethanol was added. The weight was increased by 5.93%. Next, a moisture barrier forming solution containing 14.49 g of Opadry, and 76.07 g of pure water was added. The weight was increased by 4.62%. After that, an enteric coating solution containing 27.16 g of hydroxypropyl methylcellulose acetate succinate (HPMCAS, AS-LG), 2.17 g of triethyl citrate, 1.36 g of titania, 4.96 g of talc, 60.17 g of absolute ethanol, and 243.08 g of pure water was added, and the enteric coating accounted for 8.28% by weight. Thus, TBN enteric-coated tablets were obtained.

### Example 22

TBN (API), mannitol, Crospovidone, sodium bicarbonate and magnesium stearate were sieved through a 40-mesh screen for later use. 100 g of TBN (API), 100 g of mannitol, 10 g of Polyvidone K30, 25 g of Crospovidone, and 60 g of sodium bicarbonate were weighed, and mixed for 30 min in a hopper mixer in the laboratory. Then, 1 g of magnesium stearate was added and mixed for another 1 min. The resulting material was directly tableted.

Then the tablet cores were added to a high-efficiency coating machine, and the prepared isolation coating solution containing 6.61 g of hydroxypropyl cellulose, 6.61 g of talc, and 101.37 g of absolute ethanol was added. The weight was increased by 4.67%. Next, a moisture barrier forming solution containing 15.71 g of Opadry, and 82.47 g of pure water was added. The weight was increased by 5.07%. After that, an enteric coating solution containing 23.70 g of Eudragit L100-55, 2.37 g of triethyl citrate, 2.37 g of titania, 11.85 g of talc, and 354.69 g of 95% ethanol was added, and the enteric layer accounted for 7.28% by weight. Thus, TBN enteric-coated tablets were obtained.

### Example 23

TBN (API), mannitol, Crospovidone, sodium bicarbonate, hydroxypropyl cellulose and magnesium stearate were sieved through a 40-mesh screen for later use. 300 g of TBN (API), 248.4 g of mannitol, 57.6 g of hydroxypropyl cellulose, 51 g of Crospovidone, and 60 g of sodium bicarbonate were weighed, and mixed for 30 min in a hopper mixer in the laboratory. Then, 3 g of magnesium stearate was added, mixed for another 1 min, and directly tableted. Then the tablet cores were added to a high-efficiency coating machine, and the prepared isolation coating solution containing 27.8 g of hydroxypropyl cellulose and 8.2 g of talc was added. The isolation layer accounted for 5% by weight. Next, an enteric coating solution containing 32.3 g of methacrylic acid-ethyl acrylate copolymer, 3.2 g of triethyl citrate, 3.2 g of titania, and 6.5 g of talc was added. The enteric layer accounted for 6% by weight. Thus, TBN enteric-coated tablets were obtained.

### Example 24

TBN (API), mannitol, Crospovidone, sodium bicarbonate, hydroxypropyl cellulose and magnesium stearate were sieved through a 40-mesh screen for later use. 300 g of TBN (API), 248.4 g of mannitol, 57.6 g of hydroxypropyl cellulose, 51 g of Crospovidone, and 60 g of sodium bicarbonate were weighed, mixed for 30 min in a hopper mixer in the laboratory, and granulated in a roller granulator. Then, 3 g of magnesium stearate was added, mixed for another 1 min, and tableted. Then the tablet cores were added to a high-efficiency coating machine, and the prepared isolation coating solution containing 27.8 g of hydroxypropyl cellulose and 8.2 g of talc was added. The isolation layer accounted for 5% by weight. Next, an enteric coating solution containing 32.3 g of methacrylic acid-ethyl acrylate copolymer, 3.2 g of triethyl citrate, 3.2 g of titania, and 6.5 g of talc was added. The enteric layer accounted for 6% by weight. Thus, TBN enteric-coated tablets were obtained.

### Example 25

TBN (API), mannitol, Crospovidone, sodium bicarbonate, hydroxypropyl cellulose and magnesium stearate were sieved through a 40-mesh screen for later use. 300 g of TBN (API), 248.4 g of mannitol, 57.6 g of hydroxypropyl cellulose, 51 g of Crospovidone, and 60 g of sodium bicarbonate were weighed, mixed for 30 min in a hopper mixer in the laboratory, and granulated in a roller granulator. Then, 3 g of magnesium stearate was added, mixed for another 1 min, and tableted. Then the tablet cores were added to a high-efficiency coating machine, and the prepared isolation coating solution containing 27.8 g of hydroxypropyl cellulose and 8.2 g of talc was added. The isolation layer accounted for 5% by weight. Next, an enteric coating solution containing 16.2 g of methacrylic acid-ethyl acrylate copolymer, 1.6 g of triethyl citrate, 1.6 g of titania, and 3.3 g of talc was added. The enteric layer accounted for 3% by weight. Thus, TBN enteric-coated tablets were obtained.

### Performance tests

(1) The tablet cores prepared in Example 1 and Example 2 were allowed to store under high humidity and high temperature conditions for 10 days to investigate their stability. For specific experimental procedures, refer to the Technical Guidelines for the Stability of Chemical Drugs. The results are shown in Table 1 below.

**Table 1. Stability test results of TBN tablet cores under high temperature and high humidity conditions**

| Prescription | Storage conditions | Time (day) | RRT (%) | | | Total impurity (%) |
|---|---|---|---|---|---|---|
| | | | 1.21 (Pyrazine-carbaldehyde) | 1.73 | 1.88 | |
| Example 1 | Frozen and sealed | 0 | 0.03 | 0.04 | 0.03 | 0.19 |
| | 60°C RH75% | 10 | 0.01 | 0.04 | 0.03 | 0.18 |
| | Room temperature, RH92.5% | 10 | 0.07 | 0.04 | 0.03 | 0.24 |
| Example 2 | Frozen and sealed | 0 | 0.03 | 0.04 | 0.03 | 0.18 |
| | 60°C/RH75% | 10 | 0.01 | 0.04 | 0.03 | 0.20 |
| | Room temperature, RH92.5% | 10 | 0.06 | 0.04 | 0.03 | 0.23 |

| | | | | | | |
|---|---|---|---|---|---|---|
| Note: Only individual impurities of ≥0.03% are shown in this table. | | | | | | |

The results show that related substances in the examples have no significant increase, and the stability is good.
(2) The performance indicators of the prepared tablet cores with the compositions of Example 5 and Example 6 were tested. For specific experimental procedures, refer to Guidelines for Pharmaceutical Manufacturing, Chinese Pharmacopeia 2015 Edition. The results are shown in Table 2 below.

**Table 2. Main observation indicators and results**

| Observation indicators | Example 5 | Example 6 |
|---|---|---|
| Compressibility | Good | Good |
| Cracking or not | No | No |
| Fluidity | Good | Good |
| Hardness | 50-60 N | 60-70 N |
| Disintegration time | 5-7 min | 6-8 min |
| Punch sticking | No | No |
| Friability | 0.61% | 0.22% |

The results show that increasing the amount of binder has little effect on the disintegration time.
(3) The performance indicators of the prepared tablet cores with the compositions of Example 7 and Example 9 were tested. For specific experimental procedures, refer to Guidelines for Pharmaceutical Manufacturing, Chinese Pharmacopeia 2015 Edition. The results are shown in Table 3 below.

**Table 3. Hardness and results**

| Name | Example 7 | Example 9 |
|---|---|---|
| Hardness (N) | 130-150 (main pressure: 15 KN) | 126-158 (main pressure: 13-18 KN) |
| Preparation process | Direct tableting | Dry granulation |

The results show that the composition of the present invention can be prepared by direct tableting, or by dry granulation.
(4) The disintegration time of the tablet core prepared in Examples 10-12 was determined. For specific experimental procedures, refer to Guidelines for Pharmaceutical Manufacturing, Chinese Pharmacopeia 2015 Edition. The results are shown in Table 4 below.

**Table 4. Disintegration time and results**

| Name | Example 10 | Example 11 | Example 12 |
|---|---|---|---|
| Disintegration time (min) | 15-17 | 14-17 | 12-13 |

The results show that all the tablets can meet the requirements of medicinal use.
(5) The enteric-coated tablets prepared in Example 13 were allowed to store under high humidity and high temperature conditions for 10 days to investigate their stability. The specific test method is the same as that shown in the performance test (1), and the results are shown in Table 5 below.

**Table 5. Stability results of TBN tablets under high temperature and high humidity conditions**

| Prescription | Storage conditions | Time (day) | RRT | | Total impurity (%) |
|---|---|---|---|---|---|
| | | | 1.21 (Pyrazine-carbaldehyde) | 1.71 | |
| Example 13 | Frozen and sealed | 0 | 0.07 | 0.00 | 0.07 |
| | 60°C RH75% | 10 | 0.37 | 0.06 | 0.44 |

The results show that the composition of the isolation layer of the present invention can effectively improve the stability of enteric-coated tablets.
(6) The dissolution rate of the TBN enteric-coated tablets in Examples 15 and 16 were tested. For specific experimental procedures, refer to the "Technical Guidelines for the Dissolution Test of Common Oral Solid Preparations". The results are shown in Table 6.

**Table 6. Dissolution rate test**

| Name | Example 15 | Example 16 |
|---|---|---|
| Buffer phase: The dissolution rate Q is equal to 70% in 60 minutes. | 100 | 101 |

The results show that the weight increased by coating the isolation layer has no significant effect on the dissolution rate.
(7) The TBN enteric-coated tablets in Examples 17 and 18 were allowed to store at 50°C and RH75% for 60 days and 40°C and RH75% for 90 days to investigate their stability. The related substances were tested as shown in Table 7.

**Table 7. Test data of related substances in TBN enteric-coated tablets**

| Prescription | Specification | Storage conditions | Time (day) | RRT | | Total impurity (%) |
|---|---|---|---|---|---|---|
| | | | | 1.21 (Pyrazine-carbaldehyde) | 1.71 | |
| Example 17 | 100 mg | Frozen and sealed | 0 | 0.04 | <0.01 | 0.04 |
| | | 50 °C RH75% | 10 | 0.07 | <0.01 | 0.07 |
| | | | 20 | 0.08 | <0.01 | 0.08 |
| | | | 30 | 0.09 | <0.01 | 0.09 |
| | | | 60 | 0.09 | <0.01 | 0.09 |
| | | 40 °C RH75% | 30 | 0.06 | <0.01 | 0.06 |
| | | | 60 | 0.07 | <0.01 | 0.07 |
| | | | 90 | 0.08 | <0.01 | 0.08 |
| Example 18 | 300 mg | Frozen and sealed | 0 | 0.04 | <0.01 | 0.04 |
| | | 50 °C RH75% | 10 | 0.06 | <0.01 | 0.06 |
| | | | 20 | 0.07 | <0.01 | 0.07 |
| | | | 30 | 0.10 | <0.01 | 0.10 |
| | | | 60 | 0.12 | <0.01 | 0.12 |
| | | 40 °C RH75% | 30 | 0.04 | <0.01 | 0.04 |
| | | | 60 | 0.08 | <0.01 | 0.08 |
| | | | 90 | 0.08 | <0.01 | 0.08 |

The results show that both the tablets in Example 17 and Example 18 have good stability.
(8) The enteric-coated tablets prepared in Examples 19-22
were allowed to store at 50°C and RH75% for 30 days and 40°C and RH75% for 90 days to investigate their stability. The test method was the same as the performance test (7). The test results are shown in Table 8.

**Table 8. Test data of related substances in TBN enteric-coated tablets**

| Prescription | Specification | Storage conditions | Time (day) | RRT | | Total impurity (%) |
|---|---|---|---|---|---|---|
| | | | | 1.21 (Pyrazine-carbaldehyde) | 1.71 | |
| Example 19 | 100 mg | Frozen and sealed | 0 | 0.04 | <0.01 | 0.04 |
| | | 50 °C RH75% | 30 | 0.09 | <0.01 | 0.09 |
| | | 40 °C RH75% | 30 | 0.06 | <0.01 | 0.06 |
| | | | 60 | 0.07 | <0.01 | 0.07 |
| | | | 90 | 0.08 | <0.01 | 0.08 |
| Example 20 | 100 mg | Frozen and sealed | 0 | 0.04 | <0.01 | 0.04 |
| | | 50 °C RH75% | 30 | 0.08 | <0.01 | 0.08 |
| | | 40 °C RH75% | 30 | 0.06 | <0.01 | 0.06 |
| | | | 60 | 0.07 | <0.01 | 0.07 |
| | | | 90 | 0.09 | <0.01 | 0.09 |
| Example 21 | 100 mg | Frozen and sealed | 0 | 0.04 | <0.01 | 0.04 |
| | | 50 °C RH75% | 30 | 0.10 | <0.01 | 0.10 |
| | | 40 °C RH75% | 30 | 0.06 | <0.01 | 0.06 |
| | | | 60 | 0.08 | <0.01 | 0.08 |
| | | | 90 | 0.08 | <0.01 | 0.08 |
| Example 22 | 100 mg | 50 °C RH75% | 0 | 0.09 | <0.01 | 0.09 |
| | | | 30 | 0.10 | <0.01 | 0.10 |
| | | 40 °C RH75% | 30 | 0.07 | <0.01 | 0.07 |
| | | | 60 | 0.09 | <0.01 | 0.09 |
| | | | 90 | 0.09 | <0.01 | 0.09 |

The results show that the related substances have no obvious changes after storage under the conditions of 40°C and RH75% for three months and 50°C and RH75% for 1 month, indicating that TBN has good stability when the above four materials are used as the enteric layer coating material.
(9) The dissolution rate of TBN enteric-coated tablets prepared according to Example 25 was tested, and the test method was the same as the performance test (6). The results are shown in Table 9.

**Table 9. Dissolution rate test**

| Name | Example 25 |
|---|---|
| Buffer phase: The dissolution rate Q is equal to 70% in 60 minutes. | 88 |
| Process | Dry granulation |

The result shows that the dissolution rate meets the requirements.
(10) Bioavailability test: Pharmacokinetic study of TBN (API) and enteric-coated tablets in beagle dogs
6 beagle dogs were randomly divided into 2 groups, each group having 3 animals. Each test animal was given with TBN (API) at a dosage of 175 mg, or 1 enteric-coated tablet with a specification of 175 mg prepared following the method in Example 17 by oral gavage. The blood sample was collected at various times (0.08, 0.25, 0.5, 1, 1.5, 2, 4, 8, 12, and 24 h), and analyzed by LC-MS/MS. A drug concentration-time curve was plotted to calculate the pharmacokinetic parameters.

**Table 10. Pharmacokinetic parameters of non-compartmental model after oral administration of enteric-coated tablets in beagle dogs**

| **Parameter** | **101** | **102** | **103** | **Average** | **Standard deviation** |
|---|---|---|---|---|---|
| AUC(0-t) (ng/mL*h) | 24338.4 | 22494.0 | 18404.9 | 21745.7 | 3036.7 |
| AUC (0-∞) (ng/mL*h) | 24338.5 | 22494.2 | 18404.9 | 21745.9 | 3036.7 |
| MRT (0-t) (h) | 1.30 | 1.57 | 1.62 | 1.50 | 0.17 |
| Vz/F (L/kg) | 0.57 | 0.62 | 0.72 | 0.64 | 0.08 |
| CLz/F (L/h/kg) | 0.58 | 0.64 | 0.83 | 0.68 | 0.13 |
| T1/2z (h) | 0.68 | 0.68 | 0.60 | 0.65 | 0.04 |
| Tmax (h) | 0.50 | 1.00 | 1.00 | 0.83 | 0.29 |
| Cmax (ng/mL) | 19688.09 | 19226.44 | 11617.97 | 16844.17 | 4531.90 |
| Body Weight (kg) | 12.40 | 12.23 | 11.52 | 12.05 | 0.47 |
| Dosing (mg/kg) | 14.11 | 14.31 | 15.19 | 14.54 | 0.57 |

**Table 11. Pharmacokinetic parameters of non-compartmental model after oral administration of TBN (API) in beagle dogs**

| **Parameter** | **201** | **202** | **203** | **Average** | **Standard deviation** |
|---|---|---|---|---|---|
| AUC(0-t) (ng/mL*h) | 5290.7 | 6935.2 | 16503.2 | 9576.4 | 6054.9 |
| AUC (0-∞) (ng/mL*h) | 5294.6 | 6935.3 | 16503.3 | 9577.7 | 6053.5 |
| MRT (0-t) (h) | 0.62 | 0.89 | 0.98 | 0.83 | 0.18 |
| Vz/F (L/kg) | 1.39 | 1.43 | 0.85 | 1.22 | 0.32 |
| CLz/F (L/h/kg) | 2.57 | 2.02 | 0.84 | 1.81 | 0.89 |
| T1/2z (h) | 0.38 | 0.49 | 0.71 | 0.52 | 0.17 |
| Tmax (h) | 0.25 | 0.25 | 0.25 | 0.25 | 0.00 |
| Cmax (ng/mL) | 7280.25 | 7291.11 | 15646.14 | 10072.50 | 4826.92 |
| Body Weight (kg) | 12.85 | 12.52 | 12.69 | 12.69 | 0.17 |
| Dosing (mg/kg) | 13.62 | 13.98 | 13.79 | 13.80 | 0.18 |

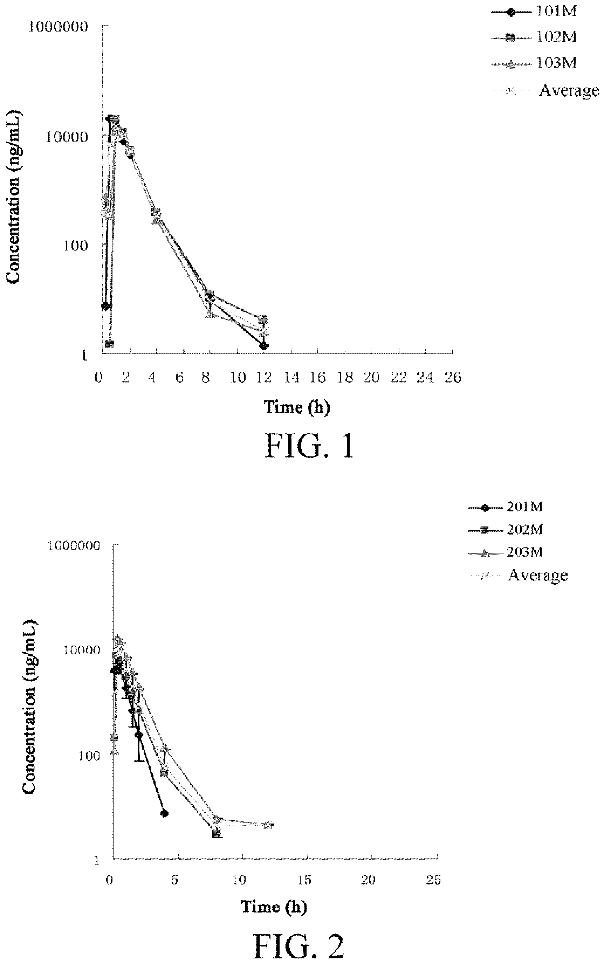

The results are shown in Tables 10 and 11 and Figs. 1 and 2. The data shows that the average Cₘₐₓ of the enteric-coated tablets in the beagle dogs is about 1.67 times the Cₘₐₓ of TBN (API) in the beagle dog, and the bioavailability of the TBN enteric-coated tablets is about 2.3 times that of the TBN (API).
(11) Pharmacokinetic study of TBN enteric-coated tablets prepared following the method described in Example 25
24 beagle dogs (male:female 1:1) were randomly divided into 4 groups (each group having 3 female and 3 male animals). Animals in group A were injected intravenously with a TBN (API) solution at a dosage of 6 mg·kg⁻¹, and animals in groups B and D were respectively given with TBN enteric-coated tablets at a dosage of 100 and 900 mg·animal⁻¹ by oral gavage. Blood was taken from the animals in group A before and 0.083, 0.25, 0.5, 1, 2, 4, 6, 8 and 24 h after administration; and blood was taken from the animals in group B and D before and 0.25, 0.5, 1, 2, 4, 6, 8, 12 and 24 h after administration. Animals in group C were given TBN enteric-coated tablets at a dosage of 300 mg·animal⁻¹ for seven consecutive days. Blood was taken from the animals in group C before and 0.25, 0.5, 1, 2, 4, 6, 8, 12 and 24 h after the first and seventh administration and before and 2 h after the second to sixth administration.

Detection and analysis by LC-MS/MS were performed and the pharmacokinetic parameters were calculated.

**Table 12. Pharmacokinetic parameters in beagle dogs intravenously injected with 6 mg·kg⁻¹ TBN**

| Parameter | C₀ (ng/ml) | t_{1/2} (h) | MRT_{0-inf} (h) | AUC_{-inf} (ng·h/ml) | CL (ml/kg·min) | VD_{SS}(L/kg) |
|---|---|---|---|---|---|---|
| Mean | 12100 | 0.292 | 0.298 | 3560 | 29.1 | 0.503 |
| SD | 1480 | 0.138 | 0.0700 | 711 | 6.03 | 0.0570 |

**Table 13. Pharmacokinetic parameters in Beagle dogs administered with TBN enteric-coated tablets at a dosage of 100, 300 and 900 mg·animal⁻¹ by oral gavage.**

| Dose | Parameter | t_{1/2} (h) | Tₘₐₓ (h) | Cₘₐₓ(ng/ml) | AUC_{0-T} (ng·h/ml) | AUC_{0-t-dose}ng·kg· (ml·mg)⁻¹ | MRT_{0-inf} (h) | F (%) |
|---|---|---|---|---|---|---|---|---|
| 100 mg· animal⁻¹ | Mean | 0.0804 | 1.25 | 4770 | 5400 | 424 | 1.50 | 71.6 |
| | SD | NA | 0.612 | 3390 | 4570 | 328 | NA | 55.4 |
| 300 mg·amimal⁻¹ (first) | Mean | 0.673 | 1.83 | 15100 | 24600 | 634 | 2.13 | 107.0 |
| | SD | 0.161 | 0.408 | 9780 | 13000 | 296 | 0.435 | 50.2 |
| 900 mg· animal⁻¹ | Mean | 0.896 | 2.50 | 96000 | 216000 | 1890 | 2.24 | 319.0 |
| | SD | 0.105 | 2.74 | 45700 | 83500 | 697 | 0.244 | 118.0 |
| 300 mg·animal⁻¹ (seventh) | Mean | 1.08 | 2.00 | 22000 | 38800 | NR | 1.86 | NR |
| | SD | 0.618 | 1.10 | 8760 | 11200 | NR | 0.361 | NR |

After intravenous administration of 6 mg TBN·kg⁻¹ in beagle dogs, the average terminal elimination half-life (t_{1/2}) is 0.292 h, the clearance (CL) is 29.1 mL·kg⁻¹·min⁻¹, the steady-state apparent volume of distribution (Vdss) is 0.503 L·kg⁻¹, and the area under the drug concentration-time curve (AUC0-inf) is 3560 ng·h·mL⁻¹. After the beagle dogs are given TBN enteric-coated tablets at a dosage of 100, 300 and 900 mg·animal⁻¹ by oral gavage, the average peak time (Tmax) is 1.25, 1.83 and 2.50 h respectively, the peak concentration (Cmax) is 4770, 15100 and 96000 ng·mL⁻¹ respectively, the AUC₀₋ₜ is 5400, 24600 and 216000 ng·h·mL⁻¹ respectively, the area under the drug concentration per dosage-time curve (AUC₀₋ₜ dose) is 424, 634 and 1890 ng·h·kg·(mL·mg)⁻¹ respectively, and the oral bioavailability (F) is 71.6%, 107.0% and 319.0% respectively. These indicate that TBN enteric-coated tablets are absorbed faster in beagle dogs after oral administration and mainly distributed in the extracellular fluid, can be quickly cleared from the body, have a bioavailability increasing with the increase in dose, and have non-linear PK characteristics especially in the high-dose group.

After a single administration of TBN enteric-coated tablets at a dosage of 100, 300, and 900 mg·animal⁻¹ to beagle dogs by oral gavage, the dose-related linear increase is basically shown in the dose range of 100-300 mg·animal⁻¹. In the dose range of 300-900 mg·animal⁻¹, more than dose-related linear increase is shown. Therefore, more than dose-related linear increase is also shown in the dose range from 100 to 900 mg·animal⁻¹.

After the beagle dogs were given TBN enteric-coated tablets at a dosage of 300 mg·animal⁻¹ by oral gavage for 7 consecutive days, the ratio of AUC₀₋ₜ after the 7th administration and the 1st administration is 1.58, and no significant drug accumulation is observation.

After intravenous administration of TBN, there is no significant difference in exposure levels (C₀, AUC₀₋ₜ) between male and female beagle dogs. Except for the low-dose administration by oral gavage, there is no significant difference in the exposure levels (Cmax, AUC₀₋ₜ) between male and female beagle dogs after administration of medium and high-dose TBN enteric-coated tablets by oral gavage.

## Claims

1. A pharmaceutical composition comprising TBN, or a salt or a hydrate thereof, the pharmaceutical composition having:
(1) a tablet core comprising the active ingredient TBN or a pharmaceutically acceptable salt or hydrate thereof and an alkalizing agent; and
(2) an enteric layer outside the tablet core, comprising an opaquer and a coating material, wherein the enteric layer accounts for 0.5-20%, preferably 1-15%, and further preferably 1-11% by weight.

2. The pharmaceutical composition according to claim 1, wherein the tablet core further comprises a binder and/or a disintegrant and/or a filler and/or a lubricant; and the enteric layer further comprises a plasticizer and/or an anti-sticking agent.

3. The pharmaceutical composition according to claim 1, wherein an isolation layer comprising a coating and an anti-sticking agent is provided between the tablet core and the enteric layer, where preferably, the isolation layer accounts for 2-15% and preferably 2-10% by weight; and preferably a moisture barrier is further provided between the isolation layer and the enteric layer, where further preferably, the moisture barrier accounts for 3-5% by weight.

4. The pharmaceutical composition according to claim 1, wherein the alkalizing agent is one or more selected from sodium bicarbonate, magnesia and magnesium carbonate, and preferably sodium bicarbonate, where preferably, the weight ratio of the active ingredient to the alkalizing agent is (90-110):(5-30) and further preferably (90-110):(10-25).

5. The pharmaceutical composition according to claim 1, wherein in the enteric layer, the opaquer is titania; and the coating material is one or more of methacrylic acid-ethyl acrylate copolymer, hydroxypropyl methylcellulose phthalate, hydroxypropyl methylcellulose acetate succinate, Eudragit^{®} L30D-55, Eudragit^{®} L100, and Eudragit^{®} NE30D, and preferably one or more of methacrylic acid-ethyl acrylate copolymer, hydroxypropyl methylcellulose phthalate or hydroxypropyl methylcellulose acetate succinate, where the weight ratio of the opaquer to the coating material is (0.5-2.5):(5-20), and preferably 1:(10-20).

6. The pharmaceutical composition according to claim 2, wherein the binder is one or more selected from hydroxypropyl cellulose, Polyvidone K30, hydroxymethyl cellulose, methyl cellulose, hydroxyethyl cellulose, carboxymethyl cellulose, and polyvinylpyrrolidone, and preferably hydroxypropyl cellulose or Polyvidone K30, where the weight ratio of the active ingredient to the binder is (90-110):(5-30), and preferably (90-110):(10-30); the disintegrant is one or more selected from Crospovidone, Croscarmellose sodium, Carboxymethyl starch sodium, sodium hydroxypropyl starch or low-substituted hydroxypropyl cellulose, and preferably Crospovidone or Carboxymethyl starch sodium, where the weight ratio of the active ingredient to the disintegrant is (90-110):(3-30), and preferably (90-110):(5-25); the filler is one or more selected from mannitol, microcrystalline cellulose, lactose, xylitol, sucrose, glucose, sorbitol, starch, pregelatinized starch, calcium sulfate, calcium carbonate, calcium hydrogen phosphate or light magnesia, preferably one or more selected from mannitol, microcrystalline cellulose, lactose or pregelatinized starch, and further preferably mannitol or pregelatinized starch, where the weight ratio of the active ingredient to the filler is (90-110):(60-200), preferably (90-110):(70-150), and further preferably (90-110):(75-140); and the lubricant is one or more selected from magnesium stearate, stearic acid, talc, hydrogenated vegetable oil, glyceryl behenate or micronized silica gel, and preferably magnesium stearate, where the weight ratio of the active ingredient to the lubricant is (90-110):(0.2-2), and preferably (90-110):(0.5-1.5).

7. The pharmaceutical composition according to claim 1, wherein the tablet core comprises the active ingredient TBN or a pharmaceutically acceptable salt or hydrate thereof, the alkalizing agent, the binder, the disintegrant, the filler and the lubricant, where the weight ratio of the active ingredient: alkalizing agent:binder:disintegrant:filler:lubricant is (90-110):(5-30):(5-30):(3-30):(60-200):(0.2-2), and preferably (90-110):(10-25):(10-30):(5-25):(70-150):(0.5-1.5).

8. The pharmaceutical composition according to claim 2, wherein the plasticizer in the enteric layer is one or more of triethyl citrate, PEG4000, PEG6000, triethyl acetyl citrate, and polysorbate-80, where preferably, the weight ratio of the opaquer to the plasticizer in the enteric layer is 1:0.5-10, and preferably 1:0.5-7; the anti-sticking agent in the enteric layer is one or more of talc, glyceryl monostearate, and micronized silica gel, where preferably, the weight ratio of the opaquer to the anti-sticking agent in the enteric layer is 1:0.5-10, and preferably 1 :0.5-5; the coating material in the isolation layer is one or more of hydroxypropyl cellulose or ethyl cellulose, and the anti-sticking agent in the isolation layer is one or more of talc, magnesia, glyceryl monostearate, and micronized silica gel, preferably talc or magnesia, and further preferably talc, where the weight ratio of the coating material to the anti-sticking agent in the isolation layer is (1-10):1, and preferably (1-5): 1; and the moisture barrier is Opadry.

9. A method for preparing a pharmaceutical composition comprising TBN, or a salt or a hydrate thereof, the method comprising the following steps:
(1) sieving the active ingredient TBN or a pharmaceutically acceptable salt or hydrate thereof, and an alkalizing agent and/or a binder and/or a disintegrant and/or a filler, directly mixing or mixing and then granulating, optionally mixing with a lubricant, and tableting to obtain tablet cores; and
(2) coating the tablet core in Step (1) with an enteric layer at 40-50°C and removing to obtain a TBN enteric-coated tablet,
wherein preferably before the coating the tablet core in Step (1) with an enteric layer at 40-50°C in Step (2), the tablet core is coated with an isolation layer at 45-65°C, removed, and then coated with the enteric layer at 40-50°C.

10. The pharmaceutical composition according to any one of claims 1 to 8 for use in the treatment of neurological diseases or cardiovascular and cerebrovascular diseases.

## Patentansprüche

1. Eine pharmazeutische Zusammensetzung, die TBN oder ein Salz oder ein Hydrat davon umfasst, wobei die pharmazeutische Zusammensetzung aufweist:
1) einen Tablettenkern, der den Wirkstoff TBN oder ein pharmazeutisch akzeptables Salz oder Hydrat davon und ein Alkalisierungsmittel umfasst, und
2) eine magensaftresistente Schicht außerhalb des Tablettenkerns, die ein Opazifierungsmittel und ein Beschichtungsmaterial umfasst, wobei die magensaftresistente Schicht 0,5-20 Gew.-%, bevorzugt 1-15 Gew.-%, und weiter bevorzugt 1-11 Gew.-% ausmacht.

2. Die pharmazeutische Zusammensetzung nach Anspruch 1, wobei der Tablettenkern ferner ein Bindemittel und/oder ein Sprengmittel und/oder einen Füllstoff und/oder ein Gleitmittel umfasst; und die magensaftresistente Schicht weiterhin ein Plastifizierungsmittel und/oder ein Antihaftmittel umfasst.

3. Die pharmazeutische Zusammensetzung nach Anspruch 1, wobei zwischen dem Tablettenkern und der magensaftresistenten Schicht eine Isolierschicht, die ein Beschichtungsmaterial und ein Antihaftmittel umfasst, vorgesehen ist, wobei vorzugsweise die Isolierschicht 2-15 Gew.-% und vorzugsweise 2-10 Gew.-% ausmacht; und vorzugsweise darüber hinaus zwischen der Isolierschicht und der magensaftresistenten Schicht eine Feuchtigkeitsbarriere vorgesehen ist, wobei weiter bevorzugt die Feuchtigkeitsbarriere 3-5 Gew.-% ausmacht.

4. Die pharmazeutische Zusammensetzung nach Anspruch 1, wobei das Alkalisierungsmittel eines oder mehrere ist, das aus Natriumbicarbonat, Magnesia und Magnesiumcarbonat, und vorzugsweise Natriumbicarbonat ausgewählt ist, wobei vorzugsweise das Gewichtsverhältnis von Wirkstoff zu Alkalisierungsmittel (90-110) : (5-30) und weiter bevorzugt (90-110) : (10-25) beträgt.

5. Die pharmazeutische Zusammensetzung nach Anspruch 1, wobei in der magensaftresistenten Schicht das Opazifierungsmittel Titandioxid ist; und das Beschichtungsmaterial eines oder mehrere von Methacrylsäure-Ethylacrylat-Copolymer, Hydroxypropylmethylcellulosephthalat, Hydroxypropylmethylcelluloseacetatsuccinat, Eudragit^{®} L30D-55, Eudragit^{®} L100 und Eudragit^{®} NE30D ist, und vorzugsweise eines oder mehrere von Methacrylsäure-Ethylacrylat-Copolymer, Hydroxypropylmethylcellulosephthalat oder Hydroxypropylmethylcelluloseacetatsuccinat ist, wobei das Gewichtsverhältnis von Opazifierungsmittel zu Beschichtungsmaterial (0,5-2,5):(5-20) und vorzugsweise 1:(10-20) beträgt.

6. Die pharmazeutische Zusammensetzung nach Anspruch 2, wobei das Bindemittel eines oder mehrere ist, das aus Hydroxypropylcellulose, Polyvidon K30, Hydroxymethylcellulose, Methylcellulose, Hydroxyethylcellulose, Carboxymethylcellulose und Polyvinylpyrrolidon und vorzugsweise Hydroxypropylcellulose oder Polyvidon K30 ausgewählt ist, wobei das Gewichtsverhältnis von Wirkstoff zu Bindemittel (90-110) :(5-30) und vorzugsweise (90-110) : (10-30) beträgt; das Sprengmittel eines oder mehrere ist, das aus Crospovidon, Croscarmellose-Natrium, Carboxymethylstärke-Natrium, Natriumhydroxypropylstärke oder niedrig substituierter Hydroxypropylcellulose und vorzugsweise Crospovidon oder Carboxymethylstärke-Natrium ausgewählt ist, wobei das Gewichtsverhältnis von Wirkstoff zu Sprengmittel (90-110) :(3-30) und vorzugsweise (90-110) :(5-25) beträgt; der Füllstoff einer oder mehrere ist, der aus Mannit, mikrokristalliner Cellulose, Lactose, Xylit, Sucrose, Glucose, Sorbit, Stärke, Quellstärke, Calciumsulfat, Calciumcarbonat, Calciumhydrogenphosphat oder leichtem Magnesia ausgewählt ist, wobei vorzugsweise einer oder mehrere aus Mannit, mikrokristalliner Cellulose, Lactose oder Quellstärke und weiter bevorzugt Mannit oder Quellstärke ausgewählt sind, wobei das Gewichtsverhältnis von Wirkstoff zu Füllstoff (90-110) : (60-200), vorzugsweise (90-110):(70-150) und weiter bevorzugt (90-110) :(75-140) beträgt; und das Gleitmittel eines oder mehrere ist, das aus Magnesiumstearat, Stearinsäure, Talkum, hydriertem Pflanzenöl, Glycerylbehenat oder mikronisiertem Silicagel und vorzugsweise Magnesiumstearat ausgewählt ist, wobei das Gewichtsverhältnis von Wirkstoff zu Gleitmittel (90-110):(0,2-2) und vorzugsweise (90-110) :(0,5-1,5) beträgt.

7. Die pharmazeutische Zusammensetzung nach Anspruch 1, wobei der Tablettenkern den Wirkstoff TBN oder ein pharmazeutisch akzeptables Salz bzw. Hydrat davon, das Alkalisierungsmittel, das Bindemittel, das Sprengmittel, den Füllstoff und das Gleitmittel umfasst, wobei das Gewichtsverhältnis von Wirkstoff:Alkalisierungsmittel:Bindemittel:Sprengmitt el:Füllstoff:Gleitmittel (90-110) : (5-30) : (5-30) : (3-30):(60-200):(0,2-2) und vorzugsweise (90-110) :(10-25):(10-30):(5-25):(70-150):(0,5-1,5) beträgt.

8. Die pharmazeutische Zusammensetzung nach Anspruch 2, wobei der Weichmacher in der magensaftresistenten Schicht eines oder mehrere von Triethylcitrat, PEG4000, PEG6000, Triethylacetylcitrat und Polysorbat-80 ist, wobei vorzugsweise das Gewichtsverhältnis von Opazifizierungsmittel zu Weichmacher in der magensaftresistenten Schicht 1:0,5-10 und vorzugsweise 1:0,5-7 beträgt; das Antihaftmittel in der magensaftresistenten Schicht eines oder mehrere von Talkum, Glycerinmonostearat und mikronisiertem Silicagel ist, wobei vorzugsweise das Gewichtsverhältnis von Opazifizierungsmittel zu Antihaftmittel in der magensaftresistenten Schicht 1:0,5-10 und vorzugsweise 1:0,5-5 beträgt; das Beschichtungsmaterial in der Isolierschicht eines oder mehrere von Hydroxypropylcellulose oder Ethylcellulose ist, und das Antihaftmittel in der Isolierschicht eines oder mehrere von Talkum, Magnesia, Glycerinmonostearat und mikronisiertem Silicagel, vorzugsweise Talkum oder Magnesia, und weiter bevorzugt Talkum ist, wobei das Gewichtsverhältnis von Beschichtungsmaterial zu Antihaftmittel in der Isolierschicht (1-10):1 und vorzugsweise (1-5):1 beträgt; und die Feuchtigkeitsbarriere aus Opadry besteht.

9. Ein Verfahren zur Herstellung einer pharmazeutischen Zusammensetzung, die TBN oder ein Salz oder ein Hydrat davon umfasst, wobei das Verfahren folgende Schritte beinhaltet:
1) Sieben des Wirkstoffs TBN oder eines pharmazeutisch akzeptablen Salzes bzw. Hydrates davon, und eines Alkalisierungsmittels und/oder eines Bindemittels und/oder eines Sprengmittels und/oder eines Füllstoffs, direktes Mischen oder Mischen und anschließendes Granulieren, wahlweise Mischen mit einem Gleitmittel, und Tablettieren, um Tablettenkerne zu erhalten; und
2) Beschichten der Tablettenkerne in Schritt 1) mit einer magensaftresistenen Schicht bei 40-50 °C und Entfernen, um eine mit einer magensaftresistenten Schicht beschichtete TBN-Tablette zu erhalten,
wobei vorzugsweise vor der Beschichtung des Tablettenkerns in Schritt (1) mit einer magensaftresistenten Schicht bei 40-50 °C in Schritt (2), der Tablettenkern mit einer Isolierschicht bei 45-65 °C beschichtet, entfernt und anschließend mit der magensaftresistenter Schicht bei 40-50 °C beschichtet wird.

10. Die pharmazeutische Zusammensetzung nach einem der Ansprüche 1 bis 8 für die Verwendung bei der Behandlung neurologischer Erkrankungen oder kardiovaskulärer und zerebrovaskulärer Erkrankungen.

## Revendications

1. Composition pharmaceutique comprenant du TBN, ou un sel ou un hydrate de celui-ci, la composition pharmaceutique comportant :
(1) un noyau de comprimé comprenant le principe actif TBN ou un sel ou un hydrate pharmaceutiquement acceptable de celui-ci et un agent alcalinisant ; et
(2) une couche entérique à l'extérieur du noyau de comprimé, comprenant un agent opaque et un matériau d'enrobage, dans laquelle la couche entérique représente 0,5-20 %, de préférence 1-15 %, et de manière davantage préférée 1-11 % en poids.

2. Composition pharmaceutique selon la revendication 1, dans laquelle le noyau de comprimé comprend en outre un liant et/ou un délitant et/ou une charge et/ou un lubrifiant ; et la couche entérique comprend en outre un plastifiant et/ou un agent anti-adhérent.

3. Composition pharmaceutique selon la revendication 1, dans laquelle une couche d'isolation comprenant un enrobage et un agent anti-adhérent est fournie entre le noyau de comprimé et la couche entérique, où de préférence la couche d'isolation représente 2-15 % et de préférence 2-10 % en poids ; et de préférence une barrière à l'humidité est en outre prévue entre la couche d'isolation et la couche entérique, où de manière davantage préférée, la barrière contre l'humidité représente 3-5 % en poids.

4. Composition pharmaceutique selon la revendication 1, dans laquelle l'agent alcalinisant est un ou plusieurs éléments choisis parmi le bicarbonate de sodium, la magnésie et le carbonate de magnésium, et de préférence le bicarbonate de sodium, où de préférence, le rapport pondéral du principe actif à l'agent alcalinisant est de (90-110):(5-30) et de manière davantage préférée de (90-110):(10-25).

5. Composition pharmaceutique selon la revendication 1, dans laquelle, dans la couche entérique, l'agent opaque est le doixyde de titane ; et le matériau d'enrobage est un ou plusieurs parmi un copolymère d'acide méthacrylique-acrylate d'éthyle, un phtalate d'hydroxypropylméthylcellulose, un acétate d'hydroxypropylméthylcellulose succinate, l'Eudragit^{®} L30D-55, l'Eudragit^{®} L100, et l'Eudragit^{®} NE30D, et de préférence un ou plusieurs parmi un copolymère acide méthacrylique-acrylate d'éthyle, un phtalate d'hydroxypropylméthylcellulose ou un acétate d'hydroxypropylméthylcellulose succinate, où le rapport pondéral de l'agent opaque au matériau d'enrobage est (0,5-2,5):(5-20), et de préférence 1:(10-20).

6. Composition pharmaceutique selon la revendication 2, dans laquelle le liant est un ou plusieurs éléments choisis parmi l'hydroxypropylcellulose, la polyvidone K30, l'hydroxyméthylcellulose, la méthylcellulose, l'hydroxyéthylcellulose, la carboxyméthylcellulose, et la polyvinylpyrrolidone, et de préférence l'hydroxypropylcellulose ou la polyvidone K30, où le rapport pondéral du principe actif au liant est de (90-110):(5-30), et de préférence de (90-110):(10-30) ; le délitant est un ou plusieurs éléments choisis parmi la crospovidone, la croscarmellose sodique, le carboxyméthylamidon sodique, amidon hydroxypropylique sodique ou l'hydroxypropyl cellulose faiblement substitué, et de préférence la crospovidone ou le carboxyméthylamidon sodique, où le rapport pondéral du principe actif au délitant est de (90-110):(3-30), et de préférence de (90-110):(5-25) ; la charge est un ou plusieurs éléments choisis parmi le mannitol, la cellulose microcristalline, le lactose, le xylitol, le saccharose, le glucose, le sorbitol, l'amidon, l'amidon prégélatinisé, le sulfate de calcium, le carbonate de calcium, l'hydrogénophosphate de calcium ou la magnésie légère, de préférence un ou plusieurs éléments choisis parmi le mannitol, la cellulose microcristalline, le lactose ou l'amidon prégélatinisé, et de manière davantage préférée le mannitol ou l'amidon prégélatinisé, où le rapport pondéral du principe actif à la charge est de (90-110):(60-200), de préférence de (90-110):(70-150), et de manière davantage préférée de (90-110):(75-140) ; et le lubrifiant est un ou plusieurs éléments choisis parmi le stéarate de magnésium, l'acide stéarique, le talc, l'huile végétale hydrogénée, le béhénate de glycéryle ou le gel de silice micronisé, et de préférence le stéarate de magnésium, où le rapport pondéral du principe actif au lubrifiant est de (90-110):(0,2-2), et de préférence de (90-110):(0,5-1,5).

7. Composition pharmaceutique selon la revendication 1, dans laquelle le noyau de comprimé comprend le principe actif TBN ou un sel ou un hydrate pharmaceutiquement acceptable de celui-ci, l'agent alcalinisant, le liant, le délitant, la charge et le lubrifiant, où le rapport pondéral du principe actif-agent alcalinisant- liant-délitant- charge- lubrifiant est de (90-110):(5-30):(5-30):(3-30):(60-200):(0,2-2), et de préférence de (90-110):(10-25):(10-30):(5-25):(70-150):(0,5-1,5).

8. Composition pharmaceutique selon la revendication 2, dans laquelle le plastifiant dans la couche entérique est un ou plusieurs parmi le citrate de triéthyle, le PEG4000, le PEG6000, l'acétylcitrate de triéthyle et le polysorbate-80, où de préférence, le rapport pondéral de l'agent opaque au plastifiant dans la couche entérique est de 1:0,5-10, et de préférence de 1:0,5-7 ; l'agent anti-adhérent dans la couche entérique est un ou plusieurs éléments parmi le talc, le monostéarate de glycéryle et le gel de silice micronisé, où de préférence, le rapport pondéral de l'agent opaque à l'agent anti-adhérent dans la couche entérique est de 1:0,5-10, et de préférence de 1:0,5-5 ; le matériau d'enrobage dans la couche d'isolation est un ou plusieurs éléments parmi l'hydroxypropylcellulose ou l'éthylcellulose, et l'agent anti-adhérent dans la couche d'isolation est un ou plusieurs éléments parmi le talc, la magnésie, le monostéarate de glycéryle et le gel de silice micronisé, de préférence le talc ou la magnésie, et de manière davantage préférée le talc, où le rapport pondéral du matériau d'enrobage à l'agent anti-adhérent dans la couche d'isolation est de (1-10):1, et de préférence de (1-5):1 ; et la barrière à l'humidité est l'opadry.

9. Méthode de préparation d'une composition pharmaceutique comprenant du TBN, ou un sel ou un hydrate de celui-ci, la méthode comprenant les étapes suivantes :
(1) tamiser le principe actif TBN ou un sel pharmaceutiquement acceptable ou un hydrate de celui-ci, et un agent alcalinisant et/ou un liant et/ou un désintégrant et/ou une charge, mélanger directement ou mélanger puis granuler, éventuellement mélanger avec un lubrifiant, et comprimer pour obtenir des noyaux de comprimés; et
(2) l'enrobage du noyau de comprimé à l'étape (1) d'une couche entérique à 40-50 °C et l'extraction de celui-ci pour obtenir un comprimé à enrobage entérique de TBN,
dans lequel, de préférence avant l'enrobage du noyau de comprimé à l'étape (1) d'une couche entérique à 40-50 °C à l'étape (2), le noyau de comprimé est enrobé d'une couche d'isolation à 45-65 °C, extrait, puis enrobé de la couche entérique à 40-50 °C.

10. Composition pharmaceutique selon l'une quelconque des revendications 1 à 8 destinée à être utilisée pour le traitement de maladies neurologiques ou de maladies cardiovasculaires et cérébrales.
